(19) Europäisches Patentamt
European Patent Office
Office européen des brevets

(11) Publication number: **0 218 441 B1**

(12) **EUROPEAN PATENT SPECIFICATION**

(45) Date of publication of patent specification: 25.04.90

(51) Int. Cl.$^5$: **A 61 K 7/00**, A 61 K 7/48

(21) Application number: 86307464.7

(22) Date of filing: 29.09.86

(54) **Cosmetic composition.**

(30) Priority: 01.10.85 JP 218921/85

(43) Date of publication of application:
15.04.87 Bulletin 87/16

(45) Publication of the grant of the patent:
25.04.90 Bulletin 90/17

(84) Designated Contracting States:
AT BE CH DE FR GB IT LI NL SE

(56) References cited:
EP-A-0 147 331
FR-A-2 243 676
FR-A-2 555 443
US-A-4 369 180

CHEMICAL ABSTRACTS, vol. 88, no. 22, May 1978, page 372, abstract no. 158324k, Columbus, Ohio, US; N.A. HARB et al.: "Inhibitory effect of 1,3-butylene glycol on microorganisms", & DRUG COSMET. IND. 1976, 118(5), 40-1, 136-7

CHEMICAL ABSTRACTS, vol. 101, 1984, page 372, abstract no. 177260q, Columbus, Ohio, US;

(73) Proprietor: LION CORPORATION
3-7, Honjo 1-chome
Sumida-ku Tokyo (JP)

(72) Inventor: Ojima, Motomu
40-1-1102, Shibuyama 1-chome
Finabashi-shi Chiba-ken (JP)
Inventor: Ishida, Keiichiro Rm. 202, Dairoku-Yano Mansion
7-17 Minamikoiwa 2-chome
Edogawa-ku Tokyo (JP)
Inventor: Tanaka, Kensuke
4-72, Toyofuta
Kashiwa-shi Chiba-ken (JP)

(74) Representative: Allam, Peter Clerk et al
LLOYD WISE, TREGEAR & CO. Norman House
105-109 Strand
London WC2R 0AE (GB)

(56) References cited:
I. RAMOS ARROCHA et al.: "Technological solution to the problem of microbial contamination in suntan lotions (emulsion)", & REV. CUBANA FARM. 1984, 18(1), 27-34

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 218 441 B1

**Description**

This invention relates generally to cosmetic compositions and, more specifically, to cosmetic compositions containing relatively large amounts of plant extracts.

Cosmetic products containing plant extracts have recently been energetically developed in order to supply consumers with natural cosmetics with high safety and in response to consumers' concern about achieving healthy skins with the aid of the natural cosmetic properties possessed by certain plant extracts.

However, since such natural active ingredients generally contain coloring or discoloring components, cosmetics with a high content of such components tend to undergo coloration or discoloration with time. Therefore, most of the commercially available cosmetics contain only a small amount of natural ingredients to avoid this coloration or discoloration problem.

By way of example, aloe plants have conspicuous effects in curing wounds, re-activating cells, and retaining heat. Therefore, remarkable cosmetic effects for roughened or chapped skins can be expected from cosmetics containing extract powder of aloe. However, in order to produce this therapeutic effect to a sufficient degree, it is necessary to use the powder extract in an amount so that the cosmetic has an aloe extract concentration similar to that of the original fresh plant. More specifically, it is necessary to mix at least 0.5 weight % of the extract powder into the cosmetic.

From US—A—4369180 a cosmetic facial preparation comprising aloe vera, cornstarch or cosmetic clay, albumin, allantoin, Vitamin A, Vitamin $D_2$ and Vitamin E is known, in which the concentration of aloe vera is between 25% and 75%. According to this prior art, it is stated that concentrated liquid aloe vera prepared in accordance therewith has a long shelf life.

However, if aloe extract powder is mixed into, for example, cream of a white or pale blue color in a concentration of greater than 0.5%, the resulting cosmetic will gradually turn to yellowish brown with the lapse of time, presumably because of chemical changes of aloe ingredients such as organic acids, salts, saccharides and amino acids. In this circumstance, presently commercially available aloe-blended cosmetics have an aloe extract content of 0.1 weight % or less, and in consequence do not possess the best possible cosmetic properties.

The present invention has been made in the light of the foregoing problems.

In accordance with the present invention, there is provided a cosmetic composition in the form of an emulsion, comprising a cosmetically-acceptable emulsified medium and:

(a) 0.5 to 10 weight % of a plant extract as an active ingredient;

(b) 0.1 to 1.0 weight % of an ester having the general formula:

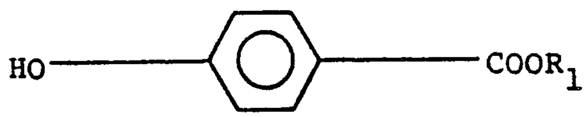

wherein $R_1$ is an alkyl group having 1 to 5 carbon atoms;

(c) 0.05 to 1.0 weight % of sorbic acid or an alkali metal salt thereof; and

(d) 0.5 to 20 weight % of an alcohol having the general formula:

$$R_2{-}(OH)_n$$

wherein $R_2$ is an alkyl group having 1 to 8 carbon atoms and n is an integer of 1 to 3.

The present invention will now be described in more detail below.

The plant extract used as an active cosmetic ingredient in the present invention is an extract from the leaves, roots, stalks or fruits of various plants such as Liliaceae plants, e.g. aloe; Cucurbitaseae plants, e.g. cucumber and snake gourd; compositae plants, e.g. pot marigold, chamomile, milfoil and arnica; and citrus plants, e.g. lemon. Extract of aloe is preferred for use herein. The plant extract may be either in the form of a liquid or a dried powder. The amount of the plant extract in the cosmetic composition is 0.5—10 weight %. When the extract is in a liquid form, the amount of the extract is calculated on the basis of its solid content.

According to the present invention, coloration or discoloration of the cosmetic composition is prevented by the inclusion of the above-defined components (b)—(d) in combination.

Component (b) is an ester of a lower alcohol with 1—5 carbon atoms with p-hydroxybenzoic acid. Illustrative of suitable esters are methyl p-hydroxybenzoate, ethyl p-hydroxybenzoate, propyl p-hydroxybenzoate, isopropyl p-hydroxybenzoate, butyl p-hydroxybenzoate and isobutyl p-hydroxybenzoate. Mixtures of two or more of these esters may be used if desired. The amount of the ester component in the cosmetic compositions should be 0.1—1.0 weight %, preferably 0.1—0.5 weight %. Whilst at least 0.1 weight % of the ester is required to achieve a useful effect in preventing unwanted coloration of the cosmetic composition, on the other hand too high a content is disadvantageous because the emulsion can than become unstable.

Component (c) is sorbic acid or an alkali metal salt thereof. The alkali metal is preferably sodium or

potassium. Component (c) is used in an amount of 0.05—1.0 weight %, preferably 0.05—0.2 weight %. An amount of component (c) below 0.05 weight % is generally insufficient to attain a useful coloration-preventing effect, but on the other hand an excess amount, above 1.0 weight % can cause the cosmetic composition to have an unpleasant odor.

Component (d) is an aliphatic alcohol having 1—8 carbon atoms. Monohydric, dihydric or trihydric alcohols may be used. Examples of suitable alcohols include methanol, ethanol, propanol, isopropanol, propylene glycol, dipropylene glycol, 1,3-butylene glycol and clycerin. At least 0.5 weight % of component (d) is generally required for attaining a useful coloration-preventing effect. An amount of component (d) above 20 weight % can adversely affect the stability of the emulsion, and the feel in use of the emulsified composition.

The components (a)—(d) are incorporated into a cosmetically-acceptable emulsified medium to form the cosmetic composition. The order of incorporation of the components (a)—(d) is not critical. Any emulsified medium conventionally used in emulsified cosmetics can be suitably used in the present invention. Both water-in-oil emulsion and oil-in-water emulsion may be used. The emulsified medium is generally composed of an oil or hydrophobic substance, water and an emulsifier.

Examples of oil or hydrophobic substance which can form an oil phase in the emulsion include waxes such as bees wax, whale wax and hydrous lanolin; hydrocarbons such as liquid paraffin, vaseline, ceresine, microcrystalline wax, squalene and pristane; higher (long chain) fatty acids such as myristic acid, stearic acid and behenic acid; esters such as isopropyl myristate, isopropyl palmitate, myristyl myristate and octyldodecyl myristate; and mixtures thereof. The oil or hydrophobic substance is generally used in an amount of 1—70 weight %, preferably 5—60 weight %. In a case where the cosmetic composition is in the form of a cream, the amount of the oil or hydrophobic substance is preferably 20—60 weight %. In the case of a lotion, the substance is preferably used in an amount of 5—25 weight %.

Any customarily employed emulsifier may be used. Anionic, cationic and nonionic surfactants are suitably used. However, the use of nonionic surfactants is preferable for reasons of safety. The emulsifier is generally used in an amount of 0.5—10 weight %, preferably 1—3 weight %.

The cosmetic composition may further include various conventionally used additives such as an antioxidant, vitamins, an ultraviolet absorber, a moisturising agent, a chelating agent, perfumes, etc. Such additives are used in such an amount as not to adversely affect the objects of the present invention, generally in an amount of 0—1 weight %.

The cosmetic composition of the present invention may be in the form of a cream or lotion and may be used, for example, as massage cream, cold cream, nutrient cream, cleansing cream or emollient cream.

The following examples will further illustrate the present invention.

## Example 1

An aqueous phase is maintained at 60°C, to which is added an oil phase, previously maintained at 70°C, with stirring by means of a homogenizer. The mixing is continued until a homogeneous emulsion is obtained. Then, a perfume is added and the emulsion is allowed to be cooled to ambient temperature with stirring by means of a paddle mixer, thereby to obtain cream. The above-mentioned aqueous phase is composed of aloe extract powder, sorbic acid, propylene glycol and water. The oil phase is composed of methyl p-hydroxybenzoate and the components shown in Table 1. The contents of respective ingredients of the cream are shown in Tables 1 and 2. The balance is water.

The aloe extract powder is a product obtained by pressing aloe plant, refining the resulting juice with activated carbon, and drying the refined extract under reduced pressure. One part by weight of aloe extract powder is obtained per 100 parts by weight of the original plant.

TABLE 1
Common Components in
Examples and Comparative Examples

| | |
|---|---|
| Liquid paraffin (#70) | 5.0 weight % |
| Squalene | 15.0 weight % |
| Cetostearyl alcohol | 5.0 weight % |
| Bees wax | 2.0 weight % |
| Glycerin monostearate | 2.0 weight % |
| Polyoxyethylene(20)sorbitan monolaurate | 2.0 weight % |

## Example 2

Example 1 is repeated in the same manner as described to obtain cream having the composition shown in Tables 1 and 2. As seen from Table 2, the composition of the cream of Example 2 is the same as that of Example 1 except that propylene glycol is replaced by ethanol of an amount shown in Table 2.

## Comparative Examples 1—5

Example 1 is repeated in the same manner as described to obtain five kinds of cream having the formulations shown in Tables 1 and 2.

Example 3

The various cream compositions obtained in Examples 1 and 2 and Comparative examples 1—5 are tested for stability during storage. Thus, each cream composition is filled in a 60 ml capped glass bottle and is allowed to stand at 40°C for 1 month. Then each cream is examined visually to check the color thereof. The results are shown in Table 2.

Example 4

Four kinds of cream compositions are prepared in the same manner as in Example 1 except that the aloe extract powder is replaced by Aloevera 200® (trademark of an aloe extract powder product of Ichimaru-Farcos Inc.), lemon extract powder, cucumber extract powder and chamomile extract powder. Stability tests are conducted in the same manner as in Example 3 to reveal that all of the cream compositions remain unchanged in color.

Table 2

Components Other Than Those Shown in Table 1 and Coloration Assessment

| | AEP*1 | MpHB*2 | SAD*3 | EOL*4 | PGL*5 | Coloration*6 |
|---|---|---|---|---|---|---|
| Example 1 | 1.0 | 0.3 | 0.1 | - | 3.0 | A |
| Example 2 | 1.0 | 0.3 | 0.1 | 1.0 | - | A |
| Comparative Example 1 | 1.0 | 0.3 | - | - | - | C |
| Comparative Example 2 | 1.0 | 0.5 | - | - | - | C |
| Comparative Example 3 | 1.0 | 0.3 | - | - | 3.0 | B |
| Comparative Example 4 | 1.0 | 0.3 | 0.1 | - | - | B |
| Comparative Example 5 | 0.1 | 0.3 | - | - | - | A |

*1 : Aloe extract powder

*2 : Methyl p-hydroxybenzoate

*3 : Sorbic acid

*4 : Ethanol

*5 : Propylene glycol

*6 : The evaluation of coloration is made according to the following ratings:

A: Almost no coloration, with a high commercial value

B: Colored in fair degree, with a low commercial value

C: Colored considerably, with no commercial value

**Claims for the Contracting States: BE CH DE FR GB IT NL SE**

1. A cosmetic composition in the form of an emulsion and containing a cosmetically-acceptable emulsified medium and a plant extract as an active ingredient, characterised in that said composition comprises:

(a) 0.5 to 10 weight % of said plant extract;
(b) 0.1 to 1.0 weight % of an ester having the general formula:

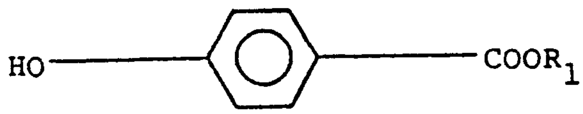

wherein $R_1$ is an alkyl group having 1 to 5 carbon atoms;
(c) 0.05 to 1.0 weight % of sorbic acid or an alkali metal salt thereof; and
(d) 0.05 to 20 weight % of an alcohol having the general formula:

$$R_2{-}(OH)_n$$

wherein $R_2$ is an alkyl group having 1 to 8 carbon atoms and n is an integer of from 1 to 3.

2. A cosmetic composition as claimed in Claim 1, characterised in that said plant extract is aloe extract powder.

3. A cosmetic composition as claimed in Claim 1 or 2, characterised in that said ester is methyl p-hydroxybenzoate, ethyl p-hydroxybenzoate, propyl p-hydroxybenzoate, isopropyl p-hydroxybenzoate, butyl p-hydroxybenzoate or isobutyl p-hydroxybenzoate.

4. A cosmetic composition as claimed in any preceding claim, characterised in that said alkali metal salt of component (c) is a sodium or potassium salt.

5. A cosmetic composition as claimed in any preceding claim, characterised in that said alcohol is methanol, ethanol, propanol, isopropanol, propylene glycol, dipropylene glycol, 1,3-butylene glycol or glycerin.

6. A cosmetic composition as claimed in any preceding claim characterised in that said component (b) is present in an amount of from 0.1 to 0.5 weight %.

7. A cosmetic composition as claimed in any preceding claim, characterised in that said component (c) is present in an amount of from 0.05 to 0.2 weight %.

**Claims for the Contracting State: AT**

1. A process for preparing a cosmetic composition in the form of an emulsion, and containing a cosmetically-acceptable emulsified medium and a plant extract as an active ingredient, characterised in that said process comprises incorporating into a cosmetically-acceptable emulsified medium the following components:

(a) 0.5 to 10 weight % of said plant extract;
(b) 0.1 to 1.0 weight % of an ester having the general formula:

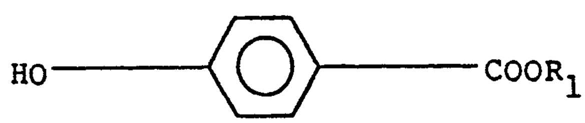

wherein $R_1$ is an alkyl group having 1 to 5 carbon atoms;
(c) 0.05 to 1.0 weight % of sorbic acid or an alkali metal salt thereof; and
(d) 0.05 to 20 weight % of an alcohol having the general formula:

$$R_2{-}(OH)_n$$

wherein $R_2$ is an alkyl group having 1 to 8 carbon atoms and n is an integer of from 1 to 3.

2. A process as claimed in Claim 1, characterised in that said plant extract is aloe extract powder.

3. A process as claimed in Claim 1 or 2, characterised in that said ester is methyl p-hydroxybenzoate, ethyl p-hydroxybenzoate, propyl p-hydroxybenzoate, isopropyl p-hydroxybenzoate, butyl p-hydroxybenzoate or isobutyl p-hydroxybenzoate.

4. A process as claimed in any preceding claim, characterised in that said alkali metal salt of component (c) is a sodium or potassium salt.

5. A process as claimed in any preceding claim, characterised in that said alcohol is methanol, ethanol, propanol, isopropanol, propylene glycol, dipropylene glycol, 1,3-butylene glycol or glycerin.

6. A process as claimed in any preceding claim, characterised in that said component (b) is incorporated in an amount of from 0.1 to 0.5 weight %.

7. A process as claimed in any preceding claim, characterised in that said component (c) is present in an amount of from 0.05 to 0.2 weight %.

**Patentansprüche für die Vertragsstaaten: BE CH DE FR GB IT NL SE**

1. Kosmetische Zusammensetzung in Form einer Emulsion und mit einem kosmetisch akzeptierbaren emulgierten Medium un einem Pflanzenextrakt als Wirkstoff, dadurch gekennzeichnet, daß die Zusammensetzung enthält:

(a) 0,5 bis 10 Gewichtsprozent des Pflanzenextraktes;

(b) 0,1 bis 1,0 Gewichtsprozent eines Esters mit der allgemeinen Formel:

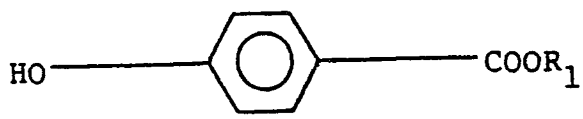

wobei $R_1$ eine Alkylgruppe mit 1 bis 5 Kohlenstoffatomen ist;

(c) 0,05 bis 1,0 Gewichtsprozent Sorbinsäure oder ein Alkalimetallsalz davon; und

(d) 0,05 bis 20 Gewichtsprozent eines Alkoholes mit der allgemeinen Formel:

$$R_2{-}(OH)_n$$

wobei $R_2$ eine Alkygruppe mit 1 bis 8 Kohlenstoffatomen und n eine ganze Zahl von 1 bis 3 ist.

2. Kosmetische Zusammensetzung nach Anspruch 1, dadurch gekennzeichnet, daß der Pflanzenextrakt Aloeextraktpulver ist.

3. Kosmetische Zusammensetzung nach Anspruch 1 oder Anspruch 2, dadurch gekennzeichnet, daß der Ester Methyl p-Hydroxybenzonat, Ethy p-Hydroxybenzonat, Propyl p-Hydroxybenzonat, Isopropyl p-Hydroxybenzonat, Butyl p-Hydroxybenzonat oder Isobutyl p-Hydroxybenzonat ist.

4. Kosmetische Zusammensetzung nach jedem der vorhergehenden Ansprüche, dadurch gekennzeichnet, daß das Alkalimetallsalz der Komponente (c) ein Natrium- oder Kaliumsalz ist.

5. Kosmetische Zusammensetzung nach jedem der vorhergehenden Ansprüche, dadurch gekennzeichnet, daß der Alkohol Methanol, Ethanol, Propanol, Isopropanol, Propylen-Glykol, Dipropylen-Glykol, 1,3-Butylen-Glykol oder Glyzerin ist.

6. Kosmetische Zusammensetzung nach jedem der vorhergehenden Ansprüche, dadurch gekennzeichnet, daß die Komponente (b) in einer Menge zwischen 0,1 bis 0,5 Gewichtsprozent vorliegt.

7. Kosmetische Zusammensetzung nach jedem der vorhergehenden Ansprüche, dadurch gekennzeichnet, daß die Komponente (c) in einer Menge zwischen 0,05 und 0,2 Gewichtsprozent vorliegt.

**Patentansprüche für den Ventragsstaat: AT**

1. Verfahren zur Herstellung einer kosmetischen Zusammensetzung in Form einer Emulsion, mit einem kosmetisch-akzeptierbaren emulgierten Medium und einem Pflanzenextrakt als Wirkstoff, dadurch gekennzeichnet, daß durch das Verfahren in das kosmetisch-akzeptierbare emulgierte Medium folgende Komponenten einarbeitet werden:

(a) 0,5 bis 10 Gewichtsprozent des Pflanzenextraktes;

(b) 0,1 bis 1,0 Gewichtsprozent eines Esters mit der allgemeinen Formel:

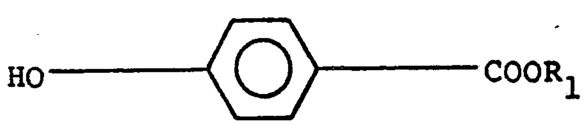

wobei $R_1$ eine Alkylgruppe mit 1 bis 5 Kohlenstoffatomen ist;

(c) 0,05 bis 1,0 Gewichtsprozent Sorbinsäure oder ein alkalisches Metallsalz davon; und

(d) 0,5 bis 20 Gewichtsprozent eines Alkohols mit der allgemeinen Formel:

$$R_2{-}(OH)_n$$

wobei $R_2$ eine Alkygruppe mit 1 bis 8 Kohlenstoffatomen ist und n eine ganze Zahl von 1 bis 3 ist.

2. Verfahren nach Anspruch 1, dadurch gekennzeichnet, daß der Pflanzenextrakt Aloeextraktpulver ist.

3. Verfahren nach Anspruch 1 oder Anspruch 2, dadurch gekennzeichnet, daß der Ester Methyl p-Hydroxybenzonat, Ethy p-Hydroxybenzonat, Propyl p-Hydroxybenzonat, Isopropyl p-Hydroxybenzonat, Butyl p-Hydroxybenzonat oder Isobutyl p-Hydroxybenzonat ist.

4. Verfahren nach jedem der vorhergehenden Ansprüche, dadurch gekennzeichnet, daß das Alkalimetallsalz der Komponente (c) ein Natrium- oder Kaliumsalz ist.

5. Verfahren nach jedem der vorhergehenden Ansprüche, dadurch gekennzeichnet, daß der Alkohol Methanol, Ethanol, Propanol, Isopropanol, Propylen-Glykol, Dipropylen-Glykol, 1,3-Butylen-Glykol oder Glyzerin ist.

6. Verfahren nach jedem der vorhergehenden Ansprüche, dadurch gekennzeichnet, daß die Komponente (b) in einer Menge zwischen 0,1 bis 0,5 Gewichtsprozent eingebracht wird.

7. Verfahren nach jedem der vorhergehenden Ansprüche, dadurch gekennzeichnet, daß die Komponente (c) in einer Menge zwischen 0,05 und 0,2 Gewichtsprozent eingebracht wird.

**Revendications pour les Etats contractants: BE CH DE FR GB IT NL SE**

1. Composition cosmétique sous forme d'une émulsion et contenant un milieu émulsionné cosmétiquement acceptable et un extrait végétal comme ingrédient actif, caractérisée en ce que la composition comprend:

(a) de 0,5 à 10% en poids de l'extrait végétal;

(b) de 0,1 à 1,0% en poids d'un ester ayant pour formule générale:

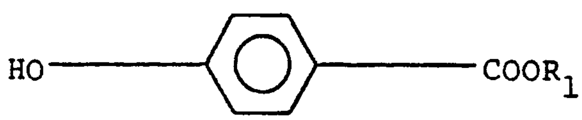

dans laquelle $R_1$ représente un groupe alkyle ayant de 1 à 5 atomes de carbone;

(c) de 0,05 à 1,0% en poids d'acide sorbique ou d'un de ses sels de métal alcalin; et

(d) de 0,05 à 20% en poids d'un alcool ayant pour formule générale:

$$R_2{-}(OH)_n$$

dans laquelle $R_2$ représente un groupe alkyle ayant de 1 à 8 atomes de carbone et n représente un nombre entier compris entre 1 et 3.

2. Composition cosmétique selon la revendication 1, caractérisée en ce que l'extrait de plante est de la poudre d'extrait d'aloès.

3. Composition cosmétique selon la revendication 1 ou 2, caractérisée en ce que l'ester est le p-hydroxybenzoate de méthyle, le p-hydroxybenzoate d'éthyle, le p-hydroxybenzoate de propyle, le p-hydroxybenzoate d'isopropyle, le p-hydroxybenzoate de butyle ou le p-hydroxybenzoate d'isobutyle.

4. Composition cosmétique selon l'une quelconque des revendications précédentes, caractérisée en ce que le sel de métal alcalin du composant (c) est un sel de sodium, ou de potassium.

5. Composition cosmétique selon l'une quelconque des revendications précédentes, caractérisée en ce que l'alcool est le méthanol, l'éthanol, le propanol, l'isopropanol, le propylèneglycol, le dipropylèneglycol, le 1,3-butylèneglycol ou la glycérine.

6. Composition cosmétique selon l'une quelconque des revendications précédentes, caractérisée en ce que le composant (b) est présent en une quantité comprise entre 0,1 et 0,5% en poids.

7. Composition cosmétique selon l'une quelconque des revendications précédentes, caractérisée en ce que le composant (c) est présent en une quantité comprise entre 0,05 et 0,2% en poids.

**Revendications pour l'Etat contractant: AT**

1. Procédé de préparation d'une composition cosmétique sous forme d'une émulsion, et contenant un milieu émulsionné cosmétiquement acceptable et un extrait végétal comme ingrédient actif, caractérisé en ce que le procédé comprend l'incorporation à un milieu émulsionné comsétiquement acceptable des composants suivants:

(a) de 0,5 à 10% en poids de l'extrait végétal;

(b) de 0,1 à 1,0% en poids d'un ester ayant pour formule générale:

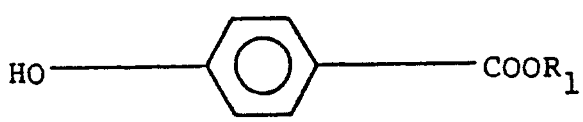

dans laquelle $R_1$ représente un groupe alkyle ayant de 1 à 5 atomes de carbone;

(c) de 0,05 à 1,0% en poids d'acide sorbique ou d'un de ses sels de métal alcalin; et

(d) de 0,05 à 20% en poids d'un alcool ayant pour formule générale:

$$R_2\text{-}(OH)_n$$

dans laquelle $R_2$ représente un groupe alkyle ayant de 1 à 8 atomes de carbone et n représente un nombre entier compris entre 1 et 3.

2. Procédé selon la revendication 1, caractérisé en ce que l'extrait végétal est de la poudre e'extrait d'aloès.

3. Procédé selon la revendication 1 ou 2, caractérisé en ce que l'ester est le p-hydroxybenzoate de méthyle, le p-hydroxybenzoate d'éthyle, le p-hydroxybenzoate de propyle, le p-hydroxybenzoate d'isopropyle, le p-hydroxybenzoate de butyle ou le p-hydroxybenzoate d'isobutyle.

4. Procédé selon l'une quelconque des revendications précédentes, caractérisé en ce que le sel de métal alcalin du composant (c) est un sel de sodium ou de potassium.

5. Procédé selon l'une quelconque des revendications précédentes, caractérisé en ce que l'alcool est le méthanol, l'éthanol, le propanol, l'isopropanol, le propylèneglycol, le dipropylèneglycol, le 1,3-butylèneglycol ou la glycérine.

6. Procédé selon l'une quelconque des revendications précédentes, caractérisé en ce qu'on incorpore le composant (b) en une quantité comprise entre 0,1 et 0,5% en poids.

7. Procédé selon l'une quelconque des revendications précédentes, caractérisé en ce que l'on incorpore le composant (c) en une quantité comprise entre 0,05 et 0,2% en poids.